(19) Europäisches Patentamt
European Patent Office
Office européen des brevets

(11) **EP 1 859 055 B2**

(12) **NEW EUROPEAN PATENT SPECIFICATION**
After opposition procedure

(45) Date of publication and mention
of the opposition decision:
**28.05.2014 Bulletin 2014/22**

(45) Mention of the grant of the patent:
**18.11.2009 Bulletin 2009/47**

(21) Application number: **06723311.4**

(22) Date of filing: **06.03.2006**

(51) Int Cl.:
*C12Q 1/68* (2006.01)

(86) International application number:
**PCT/EP2006/002159**

(87) International publication number:
**WO 2006/094798 (14.09.2006 Gazette 2006/37)**

(54) **USE OF PANEL OF PAIRS OF PRIMERS COMPLEMENTARY TO REPORTER GENES OF CELL DIFFERENTIATION**

VERWENDUNG EINER GRUPPE VON ZU REPORTERGENEN DER ZELLDIFFERENZIERUNG KOMPLEMENTÄREN PRIMERPAAREN

UTILISATION D'UN PANNEAU DE PAIRES D'AMORCES COMPLEMENTAIRES DE GENES RAPPORTEURS DE DIFFERENTIATION CELLULAIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.03.2005 DK 200500333**

(43) Date of publication of application:
**28.11.2007 Bulletin 2007/48**

(73) Proprietor: **Cellartis AB**
**413 46 Göteborg (SE)**

(72) Inventors:
• **SARTIPY, Peter**
**S-434 96 Kungsbacka (SE)**
• **NOAKSSON, Karin**
**S-443 34 Lerum (SE)**
• **ZORIC, Neven**
**411 93 Göteborg (SE)**
• **KUBISTA, Mikael**
**S-435 31 Mölnlycke (SE)**

(74) Representative: **Zacco Denmark A/S**
**Hans Bekkevolds Allé 7**
**2900 Hellerup (DK)**

(56) References cited:
**WO-A1-03//040255     US-A1- 2003 224 411**

• **CHAMBERS I ET AL: "Functional expression cloning of Nanog, a pluripotency sustaining factor in embryonic stem cells" CELL, CELL PRESS, CAMBRIDGE, NA, US, vol. 113, no. 5, 30 May 2003 (2003-05-30), pages 643-655, XP002290473 ISSN: 0092-8674**
• **STAHLBERG ANDERS ET AL: "Quantitative real-time PCR method for detection of B-lymphocyte monoclonality by comparison of kappa and lambda immunoglobulin light chain expression." CLINICAL CHEMISTRY, vol. 49, no. 1, January 2003 (2003-01), pages 51-59, XP002392048 ISSN: 0009-9147**
• **BHATTACHARYA BHASKAR ET AL: "Gene expression in human embryonic stem cell lines: unique molecular signature" BLOOD, vol. 103, no. 8, 15 April 2004 (2004-04-15), pages 2956-2964, XP002392136 ISSN: 0006-4971**
• **M. J. ABEYTA ET AL.: 'Unique gene expression signatures of independently-derived human embryonic stem cell lines' HUMAN MOLECULAR GENETICS vol. 13, no. 6, 28 January 2004, pages 601 - 608**

EP 1 859 055 B2

**Description**

**Field of invention**

The present invention relates to a kit as defined in the claims.
The panel is suitable for use e.g. in a method for rapid and sensitive determination of the state of differentiation of hBS cells. The method is based on the analysis of mRNA levels of specifically selected genes using quantitative real time PCR (QPCR).

**Background of the invention**

The successful isolation and culturing of human embryonic stem cells in 1998 marks the birth of a new era in biomedical research with wide-ranging implications for basic research, drug development and the treatment of disease. The worldwide interest in the potential of human blastocyst-derived stem cells or hBS cells is based on their unique ability to form all specific cell types and tissues found in the human body. A further important characteristic of stem cells is their capacity to reproduce themselves continuously under certain culture conditions, known as self-renewal.
Adult stem cells have been used previously for transplantation in a number of different applications, such as blood transfusion and in bone marrow, cartilage, skin and cornea transplants. However, these cells are limited in their potential to differentiate into other cell types. On the other hand, the hBS cells are pluripotent, which means that they have the potential to develop into any of the tissues and organs found in the body. However, the hBS cells are not totipotent. It is anticipated that the unique properties of hBS cells will make these cells useful in several different applications such as:

- Basic research regarding cell growth and differentiation
- Generation of new tissue for transplantation
- Reparation or replacement of damaged or diseased tissues and organs
- As a tool for novel systems in drug development
- As a model system for screening of substances in drug discovery
- As a model system for toxicity testings and developmental assays.

To generate new hBS cell lines, donated fertilized oocytes produced by in vitro fertilization for clinical purposes are used and grown to the blastocyst stage. Cells when employed according to the invention are derived from the blastocyst 4-5 days after fertilization and are therefore referred to as blastocyst-derived stem cells or hBS cells. The inner cell mass of the blastocyst is isolated by various methods including micro- or immunosurgery and plated on mitotically inactivated murine embryonic fibroblasts in a specific culture media. Under defined culture conditions undifferentiated hBS cells can be propagated indefinitely in vitro. The hBS cells can also undergo spontaneous differentiation and it is important to monitor the hBS cell culture over time. Various markers and assays can be employed to monitor hBS cells and undifferentiated hBS cells can be identified based on the following characteristics:

a. Morphology
b. Expression of surface antigen SSEA-3 and -4, TRA-1-60 and -1-81
c. No expression of surface antigen SSEA-1
d. Expression of the transcription factor Oct-4
e. Expression of alkaline phosphatase
f. Display normal karyotypes
g. Display high levels of telomerase activity
h. Generate benign teratomas in vivo, when injected into immuno-compromised experimental animals, that represent the three different germ layers
i. Having the capacity to differentiate into specialized cells *in vitro*

Visual inspection of the morphology of hBS cells in culture is a rapid but subjective characterization method, requiring extensive experience, by which it is possible to partly distinguish between undifferentiated and differentiated cells. The typical morphology of an undifferentiated hBS cell colony is recognized by tightly packed round shaped cells with a high nucleus to cytoplasm ratio.
The expression of different surface antigens (SSEA-1, -3, -4, TRA-1-60 and -1-81) is in most cases analyzed by immunohistochemistry, which gives a good indication of the phenotype of the cells although accurate quantitative data are difficult to obtain. A second drawback is that the antigens might be expressed at the cell surface even after the initiation of differentiation of the hBS cells (due to a slow turnover).

Oct-4 is a transcription factor expressed by undifferentiated hBS cells and it is known to be down-regulated upon differentiation into different precursor cells. Oct-4 can be analyzed either on the protein level (using for example immunohistochemistry or westen blot) or on the level of gene expression. However, it should be noted that Oct-4 is not exclusively expressed by undifferentiated hBS cells.

It has been shown that hBS cells display a high activity of alkaline phosphatase, but it is also known that this enzyme is also active during differentiation of hBS cells and in other cell types.

By cytogenic analysis of hBS cells, chromosomal abnormalities in the cells can be detected, which gives valuable information about the genetic integrity of the cells but it provides no indication of the state of differentiation of the cells.

High telomerase activity in cells indicates their ability to proliferate, which is a key characteristic of hBS cells. Still, some variations in telomerase activity within and between different hBS cell lines have been reported and it remains to be determined if there is a direct quantitative relationship between telomerase activity and differentiation state of the hBS cells.

Benign teratomas can be generated by injection of hBS cells into immuno-compromised experimental animals. If cells from all three embryonic germ layers are represented within the teratoma, this demonstrates the pluripotency of the injected hBS cells. This method is very time consuming and requires animal experiment, but apart from this, it is an excellent functional test of hBS cells. However, quantitative data are difficult to obtain.

Similar verification of the pluripotency of hBS cell can be performed in vitro. For such studies, hBS cell are induced to differentiate, either spontaneously or in a directed fashion, into various mature cell types representing the three embryonic germ layers. Compared to the in vivo method indicated above, this assay does not involve any animal experiments but it is still time and labour consuming and difficult to evaluate in a quantitative manner.

Genetic characterizations, such as DNA micro arrays have previously been used in order to detect genes that are up- and down regulated during differentiation (US20030224411). Major drawbacks with that technique are the lack of sensitivity and complexity if to applied as a routine analysis.

Taken together, the different characterization methods discussed above, illustrate well the status of hBS cells. However, the individual analyses each have drawbacks such as not being quantitative, requiring animal experiment, not being exclusively specific for hBS cells and some are not possible to perform in large scale. In addition, to perform all the characterization analyses mentioned above is very time consuming. Therefore, there is a need for new, simple, fast and reliable methods for the rapid and quantitative identification and characterization of hBS cells.

The expression of certain genes can be analyzed using QPCR, a sensitive method for detecting mRNA species in biological samples. The specificity of the assay depends to a large extent on the design of specific primers and other reaction conditions that are necessary for the PCR-reaction to proceed. With PCR it is possible to detect a single copy of template because of the exponential amplification during temperature cycling. A single copy of template will result in billions of copies after just 20 cycles ($2^{20}$), which can be readily detected using standard techniques. Traditionally, the obtained PCR products are detected using intercalator dyes such as ethidium bromide after separation on agarose gel by electrophoresis. Semi-quantitative information can be obtained by comparing intensity of bands on the gel, but since the amplification in many cases has entered into plateau-phase comparison between samples is difficult and inaccurate. The need to analyse all PCR products by gel electrophoresis also increases the risk of contamination when post-PCR tubes are opened.

In QPCR a fluorescent dye is included in the PCR reaction and the fluorescent signal is monitored throughout the amplification. This way one can compare how many rounds (cycles) of amplification are necessary to reach a detectable signal and is indicatory of the initial amount of template in the sample. This signal level (threshold level) is always set in the exponential phase of amplification where samples can be directly compared. Traditionally, a target gene of interest has been compared to some kind of reference gene, to take into account differences in extraction yields, reverse transcription efficiencies and differences caused by inhibitors within the samples. The difficulty has been to find suitable reference genes with stable expression throughout the study intended. In many cases housekeeping genes such as GAPDH, β-lubulin and β-actin have been chosen, but it has been demonstrated that these gense are also regulated in merry situations and can therefore cause erroneous results.

As mentioned above, there is a need for developing complementary and more specific methods for identification of undifferentisted and differentiated hBS cells. The Increasing research and development within the stem cell field also requires suitable and reliable methods for hBS cell characterization that can be used in both small and large scale. Such method can be applied for various purposes, such as:

- Quality control of hBS cell cultures
- Research tool
- Tool for toxicity testing basted on hBS cells
- Tool for evaluation of biological processes
- A model system for monitoring spontaneous as well as engineered differentiation towards different tissue types
- Tcol for hBS can based methods In drug discovery and in developmental assays monitoring early human differentiation

## Description of the invention

Despite a wide spread use of many hBS can makers (discussed above) there is a general lack of information related to how well these markers and methods actually reffect the undifferentiated and pluripotent state of hBS cells.

The present inventers have found that all known reporter genae are not equally suited for use se markers for determining the differentiation step of hBS cells, In particular, the inventors have found that it is advantageous to evaluate the expression level of a combination of reporter genes, each of which should have a similar expression profile in at least two different cell lines of the same kind of cells.

Accordingly, the invention relates to a kit as defined in claim 1.

As used herein, the term "panel" refers to a combination of pairs of primers, each pair being complementary to a reporter gene, the expression of which is to be investigated.

As used herein the term "up-regulated" is referring to a positive statistically significant (+/- 2SD) difference in gene expression. From the examples herein it is seen that e.g. AFP (alpha-feto-protein) is an example of a reporter gene, the expression of which is up-regulated.

In a similar manner, the term "down-regulated" is referring to a negative statistically significant (+/- 2SD) difference in gene expression. As seen from the examples herein e.g. Oct-4, Nanog and Cripto are reporter genes, the expression of which are down-regulated.

As used herein the term "expression profile" is referring to the expression of one or more specific genes either constantly increasing or decreasing within a certain amount of time.

The present inventors have observed that even if the expression of some reporter genes are up- or down-regulated in a certain cell line this is not necessarily a stable feature, e.g., if the expression of a reporter gene is up-regulated in a certain cell line, then it cannot be expected that the expression of the same reporter gene is up-regulated in another cell line of the same type of cells. However, with respect to the expression of certain specific reporter genes, the present inventors have found that these reporter genes behave in the same manner irrespective of the cell line (provided that it is the same kind of cells). More specifically, the inventors have found that this applies for at least two, preferably at least three, at least four, at least five or at least six cell lines, cf. the examples herein.

More specifically, the present inventors have found that the expression of the reporter genes encoding Cripto, AFP, Oct-4 and Nanog are either up- or down-regulated upon differentiation and display similar expression profiles between cell lines of the same kind of cells. The expression of other reporter genes investigated did not comply with both of these criteria.

The present invention is based on these results that provide new possibilities with respect to how to e.g. evaluate and determine the differentiation state of a cell population.

Also disclosed are

i) methods for analysing different genes, known to be expressed in undifferentiated hBS cells. Taken together, the expression levels of these genes provide valuable information about the status of the hBS cells,

ii) methods for analysing the inclusion of genes specifically expressed by differentiated hBS cells. The combination of genes expressed in both undifferentiated and differentiated hBS cells makes the method extremely sensitive and accurate; an important advantage of combining these kinds of genes is the exclusion of traditional reference genes, since many of these are known to be regulated as well and can therefore cause erroneous results,

iii) use of a mathematical model, which is based on the ratio of geometric averages of the multiple genes analysed. With this model it is possible to determine an expression index based on the relative mRNA levels two or more key genes in a biological sample.

iv) use of the expression indices calculated to compare the differentiation state of different populations of hBS cells.

With the methods described herein using the knowledge of specific reporter genes, it is possible to distinguish, with high resolution, between undifferentiated and differentiated hBS cells. Undifferentiated hBS cells will result in a high expression index, whereas differentiated hBS cells will result in a low expression index.

In the methods described herein a panel is used. The panel is used in assays for real-time PCR analysis.

Depending on the specific use of the panel, a panel described herein may be

i) a panel, wherein the expression of one of the at least two reporter genes is up-regulated upon differentiation of cells,

ii) a panel, wherein the expression of one of the at least two reporter gene is down-regulated upon differentiation of cells,

iii) a panel, wherein one of the at least two pairs of primers is complementary to a reporter gene, the expression of which is up-regulated, and another of the at least two pair of primers is complementary to a reporter gene. the expression of which is down-regulated,

iv) a panel, which comprises three or more pairs of primers, which are complementary to three or more reporter genes, wherein the expression of the three or more reporter genes are

i) either up- or down-regulated upon cell differentiation, and
ii) display a similar expression profile in at least two different cell lines of the same kind of cells,

v) a panel, which comprises four or more pairs of primers, which are complementary to four or more reporter genes, wherein the expression of the four or more reporter genes are

i) either up- or down-regulated upon cell differentiation , and
ii) display a similar expression profile in at least two different cell lines of the same kind of cells.

vi) a panel, which comprises at least two pairs of primers, which are complementary to at least two reporter genes, wherein the expression profile of the at least two reporter genes, particularly when viewed at the respective expression indices as calculated for individual cell lines, is useful to distinguish hBS cells based on their differentiation state.

More specifically, a panel described herein is a panel, wherein the at least two pairs of primers are selected from pairs of primers that are complementary to two or more of the reporter genes AFP, Cripto, Oct-4 and Nanog. For example one of the at least two pairs of primers, such as, e.g. three pairs of primers or four pairs of primers, may be complementary to AFP whereas one or more of the other at least two pairs of primers is complementary to a reporter gene, the expression of which is down-regulated upon cell differentiation. Such reporter gene, the expression of which is down-regulated upon cell differentiation, may be selected from the group consisting of Cripto, Oct-4 and Nanog.

Also disclosed is a panel comprising two pairs of primers that are complementary to two reporter genes, the expression of which are up-regulated and down-regulated upon cell differentiation, respectively. These two pairs of primers may be complementary to the reporter genes AFP and Cripto, respectively.

Also disclosed is a method of analyzing in concert the expression of two or more different genes in hBS cells, using QPCR, comprising the following steps:

i. Selection of stable reporter genes
ii. Primer design
iii. QPCR
iv. Quantification
v. Comparison

*Selection of stable reporter genes*

Genes suitable as markers for undifferentiated hBS cells or their differentiated progenies can be selected by studying genetic profiles resulting from e.g. macro scale analysis and literature in the field, such as relevant public or private expression libraries. Results from PCR analysis (see Ct-values) further show that some genes are better suited as reporter genes than others. A reporter gene should have the following basic criteria: a) It should be either up or down regulated upon differentiation of the hBS cells and b) it should display a similar expression profile in several (e.g. more than two different hBS cell lines). Furthermore, when selecting a panel of reporter genes it is of importance that the combination of genes is chosen appropriately in order to maximize the sensitivity of the assay. Here we show that the combination of Cripto and AFP is clearly sufficient to discriminate between undifferentiated hBS cells and early derivatives thereof as further described in Reference Example 9 and 10. Other combinations of potential reporter genes may not provide the same sensitivity. In addition, by adding several appropriate reporter genes (such as Oct-4 and Nanog) further robustness can be added to the assay. On the other hand, if such reporter genes are selected arbitrarily without prior testing there is a significant risk that the sensitivity and robustness of the assay is compromised. The combination of genes in the present invention includes Oct-4, Nanog, Cripto, and AFP.

A panel could comprise primers complementary to other genes as those listed below, provided that they are

i) either up- or down-regulated upon cell differentiation , and
ii) display a similar expression profile in at least two different cell lines of the same kind of cells.

Examples of genes known to be down-regulated upon cell differentiation and as such may be used as reporter genes include the following genes: Tert (telomerase reverse transcriptase), Sox-2 (homeobox-domain transcription factor 2), Rex-1 (a zinc finger protein), Utf-1 (a transcriptional co-activator), Dppa-5 (developmental-pluripotency associated 5), Cx-43 and Cx-45 (gap junction proteins connexin-43 and -45), ABCG-2.

Examples of genes known to be up-regulated upon cell differentiation and as such may be used as reporter genes include the following genes:_the early endodermal markers Brachyury, Sox-17, Goosecoid, Mixl-1, Foxa-2 (sometimes also referred to as HNF-3beta), and CXCR-4, the early mesodermal markers Brachyury (mesendodermal), Meox-1, Fox-F1, KDR, and Bmp-4 and early ectodermal markers such as Sox-1 and Zic-1.

The expression of genes can be analyzed in all different combinations such as; Oct-4/AFP, Cripto/AFP, Nanog/AFP, Oct-4*Nanog*Cripto/AFP, Oct-4*Cripto/AFP, Nanog*CriptolAFP, or Oct-4*Nanog/AFP.

Disclosed herein is a panel comprising at least two pairs of primers that are complementary to at least two different reporter genes, wherein the expression of one of the at least two reporter genes is up-regulated upon differentiation of cells and/or wherein the expression of one of the at least two reporter genes is down-regulated upon differentiation of cells.

As mentioned in the above, evaluation of the expression of more than two reporter genes improves the assay for determination of differentiation state disclosed herein, why another embodiment of the present disclosure relates to a panel comprising three or more, such as, e.g., four or more, pairs of primers, which are complementary to three or more, such as, e.g. four or more, reporter genes, wherein the expression of the three or more reporter genes are

i) either up- or down-regulated upon cell differentiation, and
ii) display a similar expression profile in at least two different cell lines of the same kind of cells.

Also disclosed is a panel comprising at least two reporter genes, each of which displaying a similar expression profile in at least three, such as, e.g., at least four, at least five or at least six different cell lines of the same kind.

It is disclosed herein, that the expression levels of the reporter genes encoding AFP, Cripto, Oct-4 and Nanog are particularly suitable for evaluation of differentiation state of hBS cells. The Ct values obtained by QPCR in five different cell lines indicated consistent either up- or down-regulation and similar expression profiles of these reporter genes from cell line to cell line, which criteria were not met by the Ct values obtained for the reporter genes encoding DNMT3B, Sox-2, Gdf-3 and β-III-tubulin.

Also disclosed is a panel of pairs of primers complementary to at least two pairs of primers selected from pairs of primers that are complementary to two or more of the reporter genes AFP, Cripto, Oct-4 and Nanog.

QPCR

RNA for use in as template in QPCR can be obtained from pluripotent/undifferentiated BS cells, especially hBS cells. These BS cells can be obtained from a BS cell line, especially an hBS cell line.

A hBS cell line for use to obtain RNA is described in WO 03/055992.

The cells may be in any stage of development. The hBS cells may be cultured with or without supporting material including, but not limited to mouse EF cells, human EF cells and extracellular matrices.

Such suitable hBS cells have at least one of the following properties

a) exhibit proliferation capacity in an undifferentiated state for more than 12 month when grown on mitotically inactivated embryonic feeder cells or under feeder free growth conditions,
b) exhibit and maintain their karyotype, being normal as well as abnormal,
c) maintain potential to develop into derivatives of all types of germ layers both *in vitro* and *in vivo*,
d) exhibit at least two of the following markers Oct-4, alkaline phosphatase, the carbohydrate epitopes SSEA-3, SSEA-4, TRA-1-60, TRA-1-81, and the protein core of a keratin sulphate/chondroitin sulphate pericellular matrix protetoglycan recognized by the monoclonal antibody GCTM-2,
e) do not exhibit molecular markers SSEA-1 or other differentiation markers, and
f) retain their pluripotency and form teratomas *in vivo* when injected into immuno-compromised mice,
g) are capable of differentiating into derivatives of all three germ layers.

The hBS cells may have all the properties mentioned above.

The expression of genes may be quantified using QPCR. The buffers and reagents used are, but not limited to, as described in "General description of the procedure". Similar solutions and reagents may be used. QPCR reactions are preferably run in the Rotorgene 3000 (Corbett Research) but can also be run in any type of real time PCR machine. The temperature of the denaturation/activation step is normally 95°C, the denaturation step is normally 95°C. the annealing step is normally 60°C and the elongation step is normally 72°C. The time period of the denaturation/activation step is normally 3 min, the denaturation step is normally 20 s, the annealing step is normally 20 s and the elongation step is normally 20 s. The total number of cycles is normally 45. It is emphasized that the skilled artisan will know that temperatures, time intervals and number of cycles can be varied.

*Expression index*

The expression index, I, (also called ratio) may be determined by calculating the ratio of expression of different genes in one sample. The mathematical model used is preferably, but not limited to (Eq. 1):

$$Ratio = K_{RS} \frac{\sqrt[n]{\left(1+E_{gene(1)}\right)^{CT_{gene(1)}} \cdot \left(1+E_{gene(2)}\right)^{CT_{gene(2)}} \cdot \ldots \cdot \left(1+E_{gene(n)}\right)^{CT_{gene(n)}}}}{\sqrt[m-n]{\left(1+E_{gene(n+1)}\right)^{CT_{gene(n+1)}} \cdot \left(1+E_{gene(n+2)}\right)^{CT_{gene(n+2)}} \cdot \ldots \cdot \left(1+E_{gene(m)}\right)^{CT_{gene(m)}}}}$$

E is the PCR efficiency, Ct is the threshold cycle, and n and (m-n) are the numbers of genes that are up and down-regulated, respectively, upon differentiation of hESC. The PCR efficiencies can be evaluated from dilution series of purified PCR products. $K_{RS}$ is the relative sensitivity constant that, among other things, accounts for the differences in templates' fragment lengths. It does not affect relative comparisons of samples. This mathematical model is derived from the equation where 2 genes can be compared (Eq. 2):

$$\frac{N_{0,gene1}}{N_{0,gene2}} = K_{RS} \frac{\left(1+E_{gene2}\right)^{CT_{gene2}}}{\left(1+E_{gene1}\right)^{CT_{gene1}}}$$

where No is the initial amount of template copies.

In order to assess variations in state of differentiation between at least two cell lines the expression of at least two reporter genes can be compared for at least two cell lines by obtaining the ratio between their respective expression indexes.

Since evaluation of differentiation state by calculation of the Expression index can be performed using QPCR data for at least two reporter genes of which one is up-regulated and the other is down-regulated, different combinations of suitable reporter genes can be used. Disclosed herein is panel of pairs of primers, one of the at least two pairs of primers is complementary to either the AFP gene, the Cripto gene, the Oct-4 gene or the Nanog gene.

*Performance of combinations*

Performance of combinations of two or more reporter genes can be evaluated by obtaining the ratio between the expression index, I, of undifferentiated cells relative to differentiated cells. Accordingly, the performance of combinations of two or more reporter genes is evaluated by obtaining the ratio between the expression index, I, of undifferentiated cells relative to differentiated cells.

In this way it was found that if only including QPCR data for the expression of two reporter genes when calculating the Expression index, I, the best combination is AFP and Cripto. Accordingly, the present disclosure relates to a panel of primers complementary to the reporter genes AFP and Cripto, respectively. However, in the same way it was found that if to include QPCR data of a third reporter when calculating the Expression index, I, Oct-4 performs better than Nanog. Accordingly, one embodiment of the present disclosure relates to a panel of three pairs of primers, which are complementary to the reporter genes AFP, Cripto and Oct-4 and another embodiment of the present disclosure relates to a panel of four pairs of primers, which are complementary to the reporter genes AFP, Cripto, Oct-4 and Nanog.

*Primer design*

Gene sequences can be located and analysed and primers being designed and evaluated using any available software by a person skilled in the art. Genes were located and analysed using Ensembl (http://www.ensembl.org/).

Primers were designed using the Primer3 web-based software (http://frodo.wi.mit.edu/cgi-bin/primer3/primer3_www.cgi) and obtained primers evaluated using Netprimer (http://www.premierbiosoft.com/netprimer/netprlaunch.html) and Beacon Designer 2.1 (Premier Biosoft).

When possible, primers can be designed to span intron-exon boundaries to ensure that genomic DNA Is not quantified. Primers can further be designed for appropriate annealing temperatures, such as between 50°C and 70 °C , such as for 60°C annealing. To ensure specificity, all primer sequences can be searched in databases, such as the NCBI sequence database using a suitable search engine, such as BLAST (http://www.ncbi.nlm.nih.gov/BLAST/). Accessibility of the obtained primer sequences can be evaluated and resulting amplified sequence analysed using an evaluation tool such as mFold (http://www.bioinfo.rpi.edu/applications/mfoid/old/dna/form1.cgi) showing all probable secondary structures at certain conditions. The melting temperature can be analysed using a tool, such as the Oligonucleotide Properties Calculator (http://www.basic.nwu.edu/biotools/oligocalc.html).

The primers used in the present invention includes, but are not limited to:

Forward primer

Oct-4: CGAAAGAGAAAGCGAACCAG
Nanog: CCTATGCCTGTGATTTGTGG
Cripto: CTGCCCTCCCTCCTTCTAC
AFP: GAAGCAAAAGCCACAAATAACA

Reverse primer

Oct-4: AACCACACTCGGACCACATC
Nanog: AAGTGGGTTGTTTGCCTTTG
Cripto: AGGTGCTCATCCATCACAA
AFP: ACTCCCAAAGCAGCACGA

*Other aspects of the disclosure*

It is disclosed to study gene expression at any state of differentiation between undifferentiated BS cells and fully differentiated cells of endodermal, mesodermal and/or ectodermal origin, such as, e.g., hepatocytes, pancreatic cells, gut epithelial cells, cardiomyocytes, chondrocytes, osteocytes, keratinocytes, neurons, astrocytes and oligodendrocytes. The QPCR assay can be adjusted to suit different pathways. Also disclosed is a panel of pairs of primers of which at least one pair of primers is complementary to a reporter gene, the expression of which is up-regulated at any state of differentiation between undifferentiated BS cells and fully differentiated cells of endodermal, mesodermal and/or ectodermal origin, such as, e.g., hepatocytes, pancreatic cells, gut epithelial cells, cardiomyocytes, ohondrocytes, osteocytes, keratinocytes, neurons, astrocytes and oligodendrocytes.

The most important aspect is to select and validate appropriate reporter genes. The assay of choice can be exemplified by an assay that can be used to screen extrinsic factors such as substances or growth factors for their ability to induce cardiac differentiation of hBS cells. It is clear that similar assays can be designed for other purposes. The reporter genes should have either of the following criteria: a) Down regulated upon differentiation of hBS cells or b) Up regulated upon differentiation of hBS cells to cardiomyocytes. Genes fulfilling either of these criteria can be identified by screening published data or by screening relevant public or private expression libraries. Examples of genes fulfilling criteria a) are Oct-4, Nanog, and Cripto, and examples of genes fulfilling criteria b) are cardiac-specific alpha-cardiac myosin heavy chain (MHC), beta-MHC, sarcomeric myosin, alpha-actinin, and GATA4. The individual QPCR assays for each gene should initially be optimized and the PCR efficiency determined. The usefulness of the potential reporter genes should also be validated through experimental testing using QPCR and control RNA prepared from undifferentiated hBS cells, differentiated hBS cells (at various stages of differentiation), and cardiomyocytes (adult cells or cells differentiated from hBS cells). In order to design a general assay applicable for any hBS cell line it is further important to select reporter genes with expression patterns that are similar between different hBS cell lines. Once the reporter genes have been selected and validated, they can be used in the QPCR assay and the data evaluated using the Mathematical model in order to calculate the expression index, which quantitatively indicates the potency of each tested substance or factor.

Following the same approach as described above for the cardiac differentiation, assays for determination of differentiation of hBS cells to other cell types can also be performed. The only difference is that the selection of reporter genes based on critera b (see above) should be fused on the particular cell type(s) of interest. For example, genes such as neuron-specific β-III tubulin, NeuN, DoubleCortin, tyrosine hydroxylase, Map 2, NF-L, NH-H, NSE, DBH, GABA, synaptophysin, and serotonin and/or genes expressed in less differentiated neural lineages, such as Pax-6, EMX-2, Musashi can be tested and validated when differentiation of hBS cells towards neural cell types is preferable.

Furthermore, genes such as alpha-antitrypsin, liver fatty acid binding protein, cytokeratin 18, and albumin can be tested and validated when differentiation of hBS cells towards hepatocytes is preferable.

Also disclosed is the quality control of undifferentiated hBS cells in culture by performing the QPCR method described herein on a regular basis, such as between every 5th and 10th passage on one or more individual cell lines to verify culture stability, detect intra-call line variations over time and/or detect spontaneous differentiation. This can be performed by using the genes described above being up and down regulated during different lineages of induced differentiation.

Also disclosed is the detection of differences in expression profiles between different cell lines at the same time points in maybe the same culture lab, i.e. inter-cell line variations or in order to detect cell line specific reactions to e.g. culture conditions such as oxygen stress and/or medium additives, such as growth factors, support-matrices and dissociation methods by using the system herein described for induced and spontaneous differentiation.

Also disclosed is an alternative approach to many functional assays based on e.g. enzymatic activities to detect biological processes in the cells other than differentiation, such as metabolism and/or apoptosis. The assay can be designed after identification and validation of suitable reporter genes being up and down regulated during the process of interest. Up-regulated genes in the case of apoptosis could belong to the family of caspases and for metabolism belong to e.g. liver function related genes, such as different cytochromes. Down regulated genes can be any gene(s) showing a down regulated expression in the biological process of interest Once the reporter genes have been selected and validated, they can be used in the QPCR assay and the data evaluated using the Mathematical model in order to calculate the expression index, which quantitatively indicates the potency of each tested substance or factor. Through experimental testing using QPCR and control RNA prepared from different samples of untreated hBS cells and or hBS derived cells and hBS cells or hBS derived cells treated with chemical compounds at various concentrations and exposure times substance specific expression indices can be generated. To obtain a general assay applicable for any hBS cell line or hBS cell line derivative the selection of reporter genes with similar expression patterns between different hBS cell lines under slightly varied culture conditions is important.

Also disclosed is the use of such systems described above in e.g. the drug discovery process to identify differentiation inducing substances or toxic effects e.g. in candidate drugs and/or lead substances potentially on an industrial scale using high-throughput set-ups. Accordingly, a panel of pairs of primers can be used to measure the response of cells upon addition of chemicals, drug candidates and/or drugs e.g. in a read out assay of hBS cell based methods for drug discovery, in a read out assay of hBS cell based methods in toxicity testing.

Also disclosed is detecting toxic effects of chemicals after certain or discreet exposure times and doses and a subsequent analysis based on e.g. the generations of expression indices for e.g. different biological processes as described above.

Also disclosed is detecting either differentiation inducing or differentiation inhibiting effects of known or novel compounds on BS cells, i.e. *in vitro* developmental assays.

Furthermore, one aspect of the present invention relates to a kit for use in real-time PCR to assess the state of hBS cell differentiation comprising:

>   i) a panel as defined in claim 1,
>   ii) optionally a suitable PCR mix,
>   iii) optionally one or more component for reverse transcription,
>   iv) optionally, instructions for use thereof.

*Definitions and abbreviations*

As used herein, the term "blastocyst-derived stem cell" is denoted BS cell, and the human form is termed "hBS cells".

As used herein, the term "embryoid bodies" is a term that is well-defined within the field of stem cells.

As used herein the term "EF cells" means "embryonic fibroblast feeder". These cells could be derived from any mammal, such as mouse or human.

By the terms "feeder cells" or "feeders" are intended to mean cells of one type that are co-cultured with cells of another type, to provide an environment in which the cells of the second type can grow. The feeder cells may optionally be from a different species as the cells they are supporting. The feeder cells may typically be inactivated when being co-cultured with other cells by irradiation or treatment with an anti-mitotic agent such as mitomycin c, to prevent them from outgrowing the cells they are supporting.

As used herein the term "QPCR" is referring to "quantitative real-time PCR", the term "E" is referring to the PCR efficiency which can be derived as described in Example 5, the term "Ct" is referring to the cycle of threshold, terms well-known to any person skilled in the art.

As used herein the term "$K_{RS}$" is referring to the relative sensitivity constant which among other things accounts for differences of fragment lengths of templates.

As used herein a "two-gene ratio" is referring to a ratio as calculated using Equation 1 or 2 herein described by the incorporation of one gene being down regulated and one gene being up regulated. The two-gene ratio or two-gene index, used interchangeably, can be calculated for one cell line at different time points or for two or more individual cell lines at the same point enabling to compare and quantify the differentiation state.

As used herein a "three-gene ratio" is referring to a ratio as calculated using Equation 1 herein described by the incorporation of at least one gene being down regulated and at least one gene being up regulated. The three-gene ratio or three-gene index, used interchangeably, can be calculated for one cell line at different time points or for two or more individual cell lines at the same point enabling to compare and quantify the differentiation state.

*General description of the procedure*

The following description gives instructions on suitable procedures, solutions, reagents, time periods etc. However, based on the general description and guidance herein, a person skilled in the art may vary the different elements within the scope of the invention.

1. hBS cells are cultured on mouse EF cells in KO-DMEM media supplemented with 20 % serum replacement, 1 % penicillin, 1 % glutamax, 1 % nonessential amino acids, 0,1 mM β-mercaptoethanol and 4 ng/ml bFGF. The cells are passaged every 4-5 day by mechanically dissociation, using a stem cell cutting tool (Swemed Labs International, Billdal, Sweden). Spontaneously differentiated hBS colonies are obtained by culture the cells for up to 21 days without passaging. 50 % of the media is changed every $2^{nd}$-$3^{rd}$ day.

2. Undifferentiated or differentiated hBS cell colonies are cut mechanically as whole colonies, using a stem cell cutting tool (Swemed Labs International, Billdal, Sweden), washed in PBS and stored in -80°C.

3. Extraction of RNA is performed using Qiagen RNeasy Mini Kit according to the manufacturer's instructions. Quantification and quality control of RNA is performed using the NanoDrop ND-1000.

4. Reverse transcription is performed using Bio-Rad iScript First Strand Synthesis Kit (according to the manufacturer's instructions) in a Rotorgene 3000 (Corbett Research).

5. QPCR Is performed under following conditions. All genes are quantified in the same run.

| Reagen | Concentration |
| --- | --- |
| Jump Start Buffer x10 (Sigma) | 1x |
| MgCl$_2$ (Sigma-Aldrich Co, St. Louis, MO, USA) | 3mM |
| dNTP mix (Sigma-Aldrich Co. St. Louis, MO, USA) | 0.3mM |
| SYBR Green (Molecular Probes, Eugene, OR, USA) | 0.4x |
| Forward primer (MWG Biotech, Ebersberg, Tyskland) | 0.4μM |
| Reverse primer (MWG Biotech, Ebersberg, Tyskland) | 0.4μM |
| Jump Start *taq* polymeras e(Sigma-Aldrich Co, St. Louis, MO, USA) | 0.04U/μl |
| cDNA templates | 2μl |
| Final volume | 20μl |

QPCR reactions are run in a Rotorgene 3000 (Corbett Research). After an initial denaturation step of 3 min at 95°C follow 45 cycles of 20 s at 95°C. 20 s at 60°C and 20 s at 72°C. Detection of fluorescent signal is performed at 72°C In each cycle. After cycling melt curve analysis is performed ramping the temperature from 55°C to 95°C and monitoring fluorescence each 0.5°C.

6. Calculation of the expression index is performed according Eq.1.

*Figure legends*

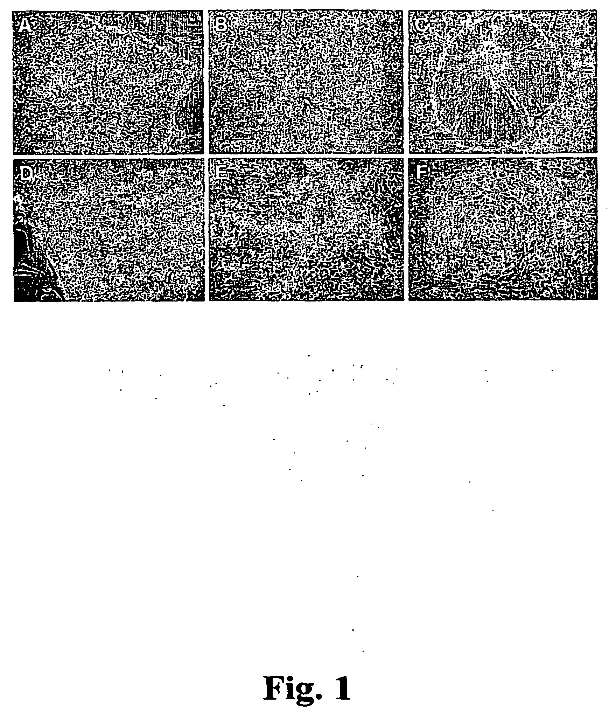

Figure 1. Representative illustrations of the morphology of undifferentiated and differentiating hBS cells (SA002 and AS034). The cells were cultured on EF cells using VitroHES medium supplemented with bFGF and passaged mechanically every 4-5 days. Extended culture of the hBS cells without passaging initiated differentiation and a mixture of early hBS cell derivatives was obtained. (A) SA002 at day 5, (B) SA002 at day 14, (C) SA002 at day 21, (D) AS034 at day 5, (E) AS034 at day 14, (F) AS034 at day 21. Magnification x100.

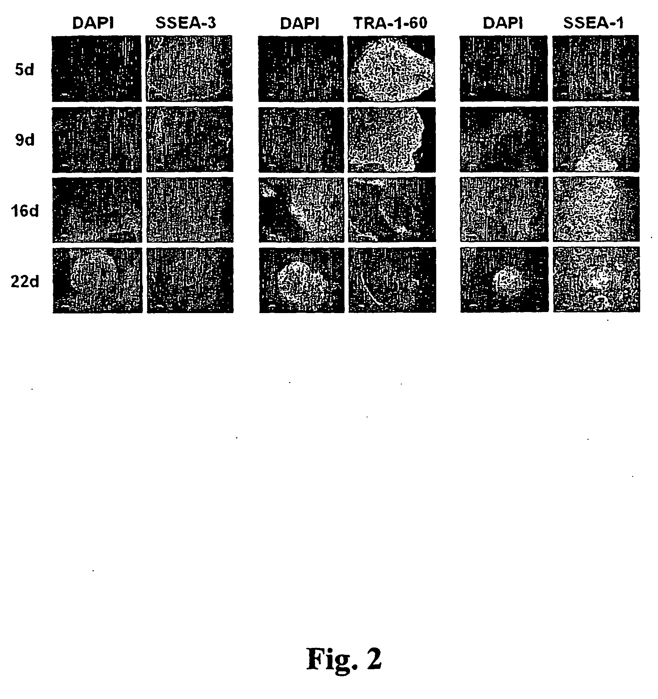

Figure 2. Undifferentiated and spontaneously differentiated hBS cell (SA181) colonies maintained on EF cells were analyzed using immunohistochemistry. The panels show representative DAPI stainings and the corresponding specific antibody staining for SSEA-3, TRA-1-60, and SSEA-1 as indicated. The cells were fixed and analyzed at the time points indicated on the left. Magnification x100.

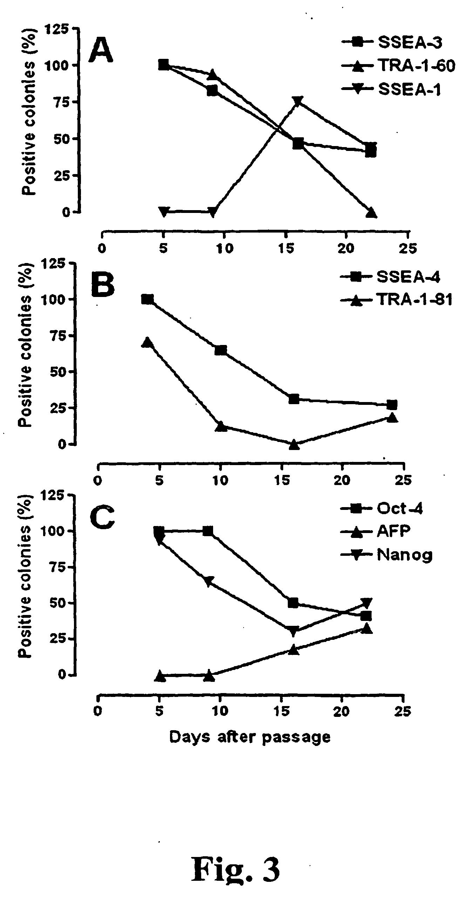

Figure 3. Semi-quantitative evaluation of the immunohistochemical staining of undifferentiated and differentiating hBS cells. A hESC colony was scored as positive if >50 % of the cells within the colony were stained by the antibody used (indicated in the figure). For each data point 12-32 individual hBS cell colonies were evaluated. (A) SA181, (B) SA202, (C) SA121.

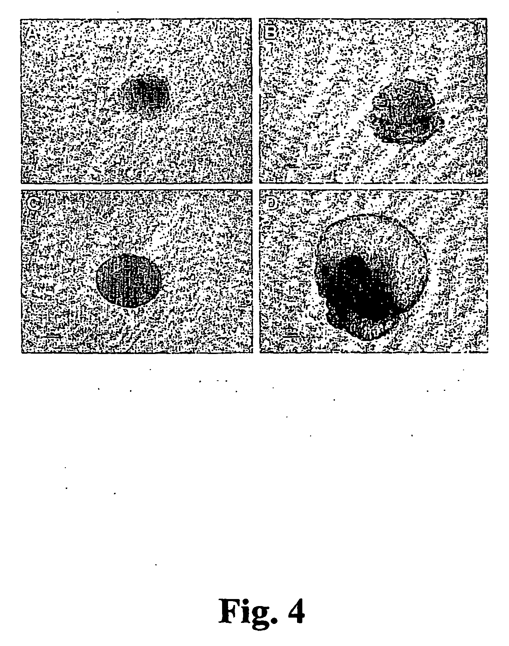

Figure 4: EB formation potential was analyzed for undifferentiated and differentiated hBS cell colonies. Three dimensional, homogenous EBs with high cell density were scored as ++ (A and C), whereas EBs attached to the surface, with cystic morphology, low cell density, or with irregular shape were scored as + (B and D). A-B, SA001; C-D, SA002.

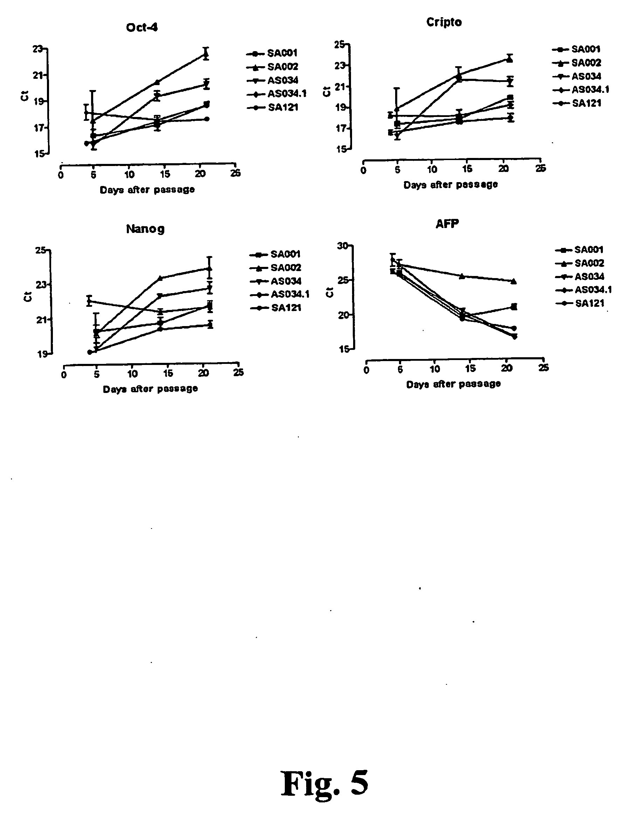

Figure 5. Ct-values obtained using OPCR. Human BS cells were cultured and differentiated on EF cells. The cells were harvested at different time points after passage as indicated on the x-axis. Total RNA was extracted and QPCR was employed to determine the gene expression levels of Oct-4. Nanog, Cripto, and AFP. Five different hBS cell lines (as indicated) were used in this set of experiments.

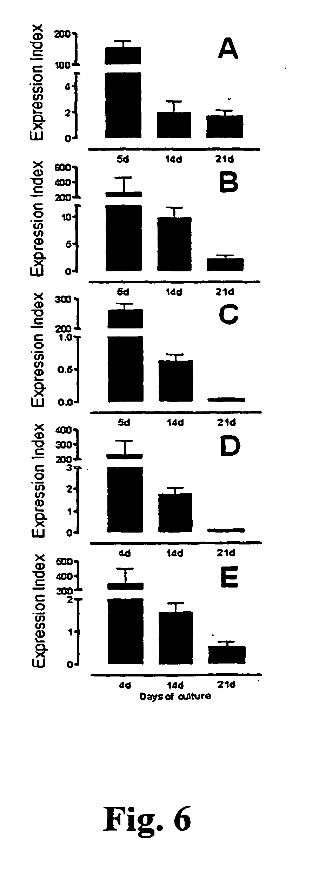

Figure 6. QPCR analysis of undifferentiated and differentiating hBS cells maintained on EF cells. The cells were harvested at the time points indicated on the x-axis. Following RNA extraction, the relative RNA levels of Oct-4, Nanog, Cripto, and AFP were determined using QPCR. The expression index was subsequently calculated using the equation Eq. 1. The data are presented as the mean plus S.D. (n=4). (A) SA001, (B) SA002, (C) AS034, (D) AS034.1, (E) SA121.

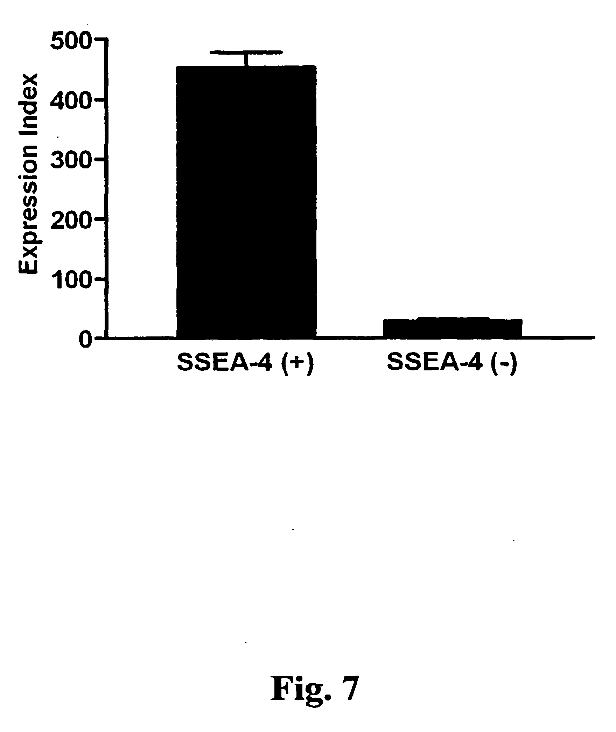

Figure 7. QPCR analysis of SSEA-4+ and SSEA-4- hBS cells. The cells (SA121) were cultured on EF cells, harvested at day 7. and fractionated using SSEA-4-antibody-coated magnetic beads. Following RNA extraction the relative RNA levels of Oct-4, Nanog, Cripto, and AFP were determined using QPCR. The expression index was subsequently calculated using equation Eq. 1. The data are presented as the mean plus S.D. (n=2).

The invention is further illustrated in the following examples, which are not intended to limit the invention in any way. Specific embodiments appear in the appended claims.

### Reference example 1

### Culture of undifferentiated and differentiated hBS cells on mouse EF cells

5 days old hBS cell colony of five different cell lines were cut in pieces with a size of 0,1-0,3 x 0,1-0,3 mm, using a stem cell cutting tool (Swemed Labs International, Billdal, Sweden) and placed on mouse EF cells in 20 dishes per cell line. Between 10 and 18 pieces were placed in each dish. The hBS cells were cultured in KO-DMEM media supplemented with 20 % serum replacement, 1 % penicillin, 1 % glutamax, 1 % nonessential amino acids, 0,1 mM β-mercaptoethanol and 4 ng/ml bFGF for 4-5 days (10 dishes per cell line), 14 days (5 dishes per cell line) and 21 days (5 dishes per cell line) respectively, without passaging. 50 % of the media were changed every 2nd-3rd day. The hBS cell colonies were cut out mechanically (as above), transferred to 1,5 ml sterile tubes and washed in PBS. Cell pellets were stored in -80 °C.
The 4-5 days old hBS cells display the morphology of tightly packed round shaped cells with a high nucleus to cytoplasm ratio, which are the typical characteristics for undifferentiated hBS cells. On the contrary, 14 and 21 days old hBS colonies displayed very different morphologies such as cystic-like structures, neuron-like structures and circle-like patterns within the colonies (see Figure 1). The colonies at day 14 and 21 after passage were considerably larger compared to after 4-5 days.

**Reference example 2**

**Immunological marker analysis of undifferentiated and differentiated hBS cells**

In order to obtain spontaneously differentiated high dense hBS cell cultures, hBS cells were cultured as in Example 1 for up to 24 days without passaging. Undifferentiated and spontaneously differentiated hBS cell colonies were fixed after 4-5 days, 9-10 days, 16 days and 22-24 days, in 4 % PFA for 15 minutes and subsequently permeabilized in 0,5 % Triton X-100 for 5 minutes. After washing in PBS and blocking in 10 % milk for 30 minutes in room temperature, the cells were incubated with primary antibodies against following antigens: SSEA-1 (1 μg/ml), SSEA-3 (1 μg/ml), SSEA-4 (1 μg/ml), TRA-1-60 (1 μg/ml), TRA-1-81 (1 μg/ml), Oct-4 (1 μg/ml), Nanog (1 μg/ml), and AFP (1 μg/ml), for 20h in +4 °C. The cells were then washed in PBS and incubated with FITC- or Cy3-conjugated secondary antibodies mixed with DAPI (0,5 μg/ml, for nuclei visualization) for 1 h in room temperature, followed by washing in PBS and mounting. The stained hBS cell colonies were visualized in an inverted fluorescence microscope. For semi-quantitative evaluation, an hBS cell colony was scored as positive if >50 % of the cells in the colony were stained by the antibodies used.

Semi-quantitative marker analysis of hBS cells indicated that the expression of SSEA-3, SSEA-4, TRA-1-60, TRA-1-81, Oct-4, and Nanog was decreased in spontaneously differentiated hBS cell colonies compared to undifferentiated cells. The expressions of these antigens never disappeared completely within the time for differentiation used in this study, though. Notably, the expression levels were still high even after 9-10 days, even though some morphology changes of the colonies were observed at that time point. It was also shown that SSEA-1 and AFP were not expressed in undifferentiated hBS cell colonies but after 16 days these antigens appear in differentiated colonies (see figure 2 and 3).

**Reference example 3**

**Analysis of EB formation potential of undifferentiated and spontaneously differentiated hBS cell colonies**

Undifferentiated and spontaneously differentiated hBS cells (SA001 and SA002) maintained on EF cells were mechanically dissociated into 1 x 1 mm clusters using a Stem Cell Tool. The hBS cell clusters were placed in suspension cultures and allowed to form and grow as embryoid bodies (EB) (Itskovitz-Eldor et al 2000 Mol Med) in KO-DMEM media supplemented with 20 % FBS, 1 % PEST, 1 % glutamax, 1 % nonessential amino acids and 0.1 mM β-mercaptoethanol. After 2 days the number and morphology of the EB formed were evaluated. Three dimensional, homogenous EB with high cell density were scored as ++, whereas EB that attached to the culture dish and with low cell density or with irregular shape were scored as +.
A common feature for undifferentiated hBS cells is their ability to form simple and cystic EB harboring derivatives of the three embryonic germ layers when grown in suspension. We wanted to investigate if the degree of differentiation of the hBS cells was correlated to their ability to form organized EB. To this end, hBS cell clusters from undifferentiated and differentiated colonies were manually dissected and transferred to suspension cultures. After 2 days the number of EB formed and their morphology was evaluated and graded as described in above. Representative illustrations of the appearance of the EB of the various grades are shown in Figure 4. The quantitative data are summarized in Table I (below) and demonstrated that there was a negative correlation between the level of differentiation and the cells' ability to form three dimensional, homogenous EB with high cell density. Similar results were obtained for both hBS cell lines. Cell clusters originating from undifferentiated colonies displayed a high frequency of EB formation (approximately 80%) while only 25% of the cell clusters from differentiating colonies at day 21 properly aggregated into organized EB. Following extended culture of the EB, markers indicative of cell types representing the three embryonic germ layers were detected (data not shown).

**Table I.** Evaluation of the EB formation potential by undifferentiated and differentiated hBS cells. The cells (SA001 and SA002) were mechanically dissociated and transferred to suspension cultures. After 2 days the EB formation was evaluated as described above. The figures shown in the Table below represent the ratios between the number of ++EB formed and the total number of cell clusters originally placed in the suspension cultures. The calculated percentage values are indicated within parenthesis.

|         | SA001, ++EB | SA002, ++EB |
|---------|-------------|-------------|
| 5 days  | 68/80 (85%) | 40/58 (69%) |
| 14 days | 33/80 (41%) | 24/80 (30%) |
| 21 days | 20/80 (25%) | 19/80 (24%) |

## Example 4

### Primer design

Gene sequences were located and analysed using Ensembl (http://www.ensembl.org/). Primers were designed using the Primer3 web-based software (http://frodo.wi.mit.edu/cgi-bin/primer3/primer3_www.cgi) and obtained primers were evaluated using Netprimer (http://www.premieroioso.ft.comlnetprimer/netprlaunch.html) and Beacon Designer 2.1 (Premier Biosoft). When possible, primers were designed to span intron-exon boundaries to ensure that genomic DNA was not quantified. Primers were designed for 60°C annealing. To ensure specificity, all primer sequences were searched in the NCBI sequence database using
BLAST (http://www.ncbi.nlm.nih.gov/BLAST/).
To evaluate accessibility of the obtained primer sequences the resulting amplified sequence (amplicon) was analyzed using mFold (http://www.bioinfo.rpi.edu/applications/mfold/old/dna/form1.cgi) showing all probable secondary structures at certain conditions. The amplicon melting temperature was also analyzed using the Oligonucleotide Properties Calculator (http://www.basic.nwu.edu/biotoots/oligocalc.html).
Resulting primer sequences (depicted 5'-3') and amplicon size were:

| Gene | Forward primer | Reverse primer | Amplicon size |
|---|---|---|---|
| Oct-4 | CGAAAGAGAAAGCGAACCAG | AACCACACTCGGACCACAT C | 146 |
| Nanog | CCTATGCCTGTGATTTGTGG | AAGTGGGTTGTTTGCCTTTG | 166 |
| Cripto | CTGCCCTCCCTCCTTCTAC | AGGTGCTCATCCATCACAA | 186 |
| DNMT3B | CAACAACACGCAACCAGAGA | TTCCTGCCACAAGACAAAT A | 213 |
| AFP | GAAGCAAAAGCCACAAATAAC A | ACTCCCAAAGCAGCACGA | 156 |
| Nestin | TAGAGAGCGAGCAGGAGGAG | GGGACACTGACACTCACAG AA | 223 |
| Desmin | GAGCAAAGGGGTTCTGAGGT | AGTGAGGACAGGGGTGAG G | 163 |
| Lin28 | GGGTTCGGCTTCCTGTCC | CCGCCTCTCACTCCCAATA C | 216 |
| OC90 | TGAAGTGTGTGGCTGGTCTC | CTAAGTTTGGCGGGGTCTT | 172 |
| Sox-2 | CAAAGAAAAACGAGGGAAAT G | AGTGTGGATGGGATTGGTG T | 157 |
| Gdf-3 | GCCATCAAAGAAAGGGAACA | GACTGACCGCAACACAAAC A | 174 |
| β-III-tubulin | GGGCCTTTGGACATCTCTTC | TCGCAGTTTTGACACTCCTT C | 148 |

## Example 5

### Optimization and standard curve generation

PCR was performed under standard conditions to generate PCR product PCR product of 2 pooled reactions was purified using Qiagen Qiaquick PCR Purification Kit according to the manufacturer's instructions. Elution was performed in 30 μl of EB buffer.
Determination of the DNA concentration in copies/μl of the obtained PCR product was performed on the NanoDrop ND-1000 using absorbance at 260 nm. Using the amplicon size and the absorbance the concentration could be calculated. Using the purified PCR product of each PCR system, the PCR was optimized by varying the concentration of $MgCl_2$, deoxynucleotides and primers to obtain a good PCR efficiency and as little primer-dimers as possible. All PCR reactions were performed in the Rotorgene 3000 (Cobett Research) using SYBR Green I as a reporter dye to monitor amplification during the reaction. Threshold was set above the noise and in the exponential phase of the amplification.
PCR efficiency was determined by making a serial dilution of PCR product. The Rotorgene software plots threshold cycle (the amplification round were the fluorescent signal crosses the threshold level) versus concentration and calculate the PCR efficiency, which is used in the mathematical model Eq. 1.

Cycling A.FAM/Sybr (Page 1):
R=0,99955
R^2=0,99910
M=-3,343
B=30.779
Efficiency=0,99

Melt curve analysis and gel electrophoresis was performed to ensure that the correct product had been amplified.

## Example 6

### RNA extraction and reverse transcription

Extraction of total RNA from cells was performed using Qiagen RNeasy Mini Kit according to the manufacturer's instruction. During extraction the samples were DNase treated to remove any contaminating genomic DNA. DNase treatment was performed on-column using Qiagen RNase-free DNase Kit according to instructions. RNA was eluted in 30 $\mu$l RNase-free water.

The eluted RNA was quantified and checked for quality using the NanoDrop ND-1000. Absorbance spectra from 220-750 nm were measured. Quality was determined using the $A_{280}/A_{280}$ and $A_{280}/A_{230}$ ratios and concentration was determined using $A_{260}$ and the NanoDrop software. Concentration was determined as the average of 2 separate measurements. Reverse transcription was performed on 1 $\mu$g of total RNA in a final volume of 20 $\mu$l using Bio-Rad iScript First Strand Synthesis Kit according to the manufacturer's instructions. The reverse transcription was run in the Rotorgene 3000 (Corbett Research).

The method described herein is not dependent on normalizing the input RNA amount and different RNA input for different samples has been used with success. Also, the reverse transcription reaction can be uniformly scaled down to 10 $\mu$l. No RT controls were performed on each cell line by adding water instead of RT enzyme to the reaction. This controls for the presence of genomic DNA.

Each RNA sample was reverse transcribed in duplicate to evaluate the variability of the entire method.

## Example 7

### Quantification of gene expression using QPCR

QPCR was performed under following conditions. All genes were quantified in the same run.

| Reagent | Concentration |
| --- | --- |
| Jump Start Bufferx10 (Sigma) | 1x |
| MgCl$_2$ (Sigma-Aldrich Co, St Louis, MO, USA) | 3mM |
| dNTP mix (Sigma-Aldrich Co. St. Louis, MO, USA) | 0.3mM |
| SYBR Green (Molecular Probes, Eugene, OR, USA) | 0.4x |
| Forward primer (MWG Biotech, Ebersberg, Tyskland) | 0.4$\mu$M |
| Reverse primer (MWG Biotech, Ebersberg, Tyskland) | 0.4$\mu$M |
| Jump Start *taq* polymeras e(Sigma-Aldrich Co, St. Louis, MO, USA) | 0.04U/$\mu$l |
| cDNA template | 2$\mu$l |
| Final volume | 20$\mu$l |

QPCR reactions were run in the Rotorgene 3000-(Corbett Research). After an initial denaturation/activation step of 3 min at 95°C followed 45 cycles of 20 s at 95°C, 20 s at 60°C and 20 s at 72°C. Detection of fluorescent signal was performed at 72°C in each cycle. After cycling melt curve analysis was performed by ramping the temperature from 55°C to 95°C and monitoring fluorescence each 0.5°C. Ct values were calculated by the Rotorgene software.

## Example 8

### Quantification

Quantification of gene expression was based on the Ct (threshold cycle) value for each respective sample. When comparing several genes following mathematical model was used:

$$Ratio = K_{RS} \frac{\sqrt[n]{\left(1 + E_{gene\,(1)}\right)^{CT_{gene\,(1)}} \cdot \left(1 + E_{gene\,(2)}\right)^{CT_{gene\,(2)}} \cdots \cdot \left(1 + E_{gene\,(n)}\right)^{CT_{gene\,(n)}}}}{\sqrt[m-n]{\left(1 + E_{gene\,(n+1)}\right)^{CT_{gene\,(n+1)}} \cdot \left(1 + E_{gene\,(n+2)}\right)^{CT_{gene\,(n+2)}} \cdots \cdot \left(1 + E_{gene\,(m)}\right)^{CT_{gene\,(m)}}}}$$

E is the PCR efficiency, Ct is the threshold cycle, and n and (m-n) are the numbers of genes that are up and down-regulated, respectively, upon differentiation of hBS cells.

The PCR efficiencies were evaluated from dilution series of purified PCR products. $K_{RS}$ is the relative sensitivity constant that, among other things, accounts for the differences in templates' fragment lengths. It does not affect relative comparisons of samples and was not determined.

## Reference example 9

### Selection of marker genes for undifferentiated and differentiated hBS cells

hBS cells from five different cell lines (SA001, SA002, AS034, AS034.1 and SA121) were cultured and spontaneously differentiated as described in example 1. Primer design, optimization, RNA extraction, reverse transcription and QPCR were performed as described in the examples above and the following Ct values were obtained:

| hBS cells | Oct.4 | Nanog | Cripto | DNMT3B | AFP |
|---|---|---|---|---|---|
| **SA001 5d** | 16,38 ± 0,48 | 20,29 ± 0,36 | 17,35 ± 0,23 | - | 25,99 ± 0,53 |
| **SA001 14d** | 17,15 ± 0,43 | 20,71 ± 0,31 | 17.8 ± 0,37 | - | 19,56 ± 0,21 |
| **SA001 21d** | 18,64 ⊥ 0,18 | 21,73 ⊥ 0,23 | 19,77 ⊥ 0,22 | - | 20,84 ± 0,40 |
| **SA002 5d** | 17,55 ± 2,23 | 20,15 ± 1,18 | 18,83 ± 1,9 | - | 27,16 ± 0,65 |
| **SA002 14d** | 20,40 ± 0,11 | 23,31 ± 0,05 | 21,98 ± 0,69 | **-** | |
| **SA002 21d** | 22,54 ± 0,49 | 23,87 ± 0,62 | 23,51 ± 0,35 | - | 24,53 ± 0,03 |
| **AS034 5d** | 15,69 ± 0,06 | 19.27 ± 0,33 | 16,21 ± 0,37 | - | 25,73 ± 0,19 |
| **AS03414d** | 19,34 ± 0,37 | 22,27 ± 0,11 | 21,49 ± 0,15 | - | 20,3 ± 0,36 |
| **AS03421d** | 20,20 ± 0,38 | 22.7 ± 0,33 | 21,29 ± 0,47 | - | 16,59 ± 0,07 |
| **AS034.1 4d** | 18,16 ± 0,58 | 22,05 ± 0.29 | 18,19 + 0,25 | - | 27,85 ± 0,89 |
| **AS034.1 14d** | 17,52 ± 0,32 | 21,35 ± 0,16 | 18,08 ± 0,51 | - | 19,86 ± 0,09 |
| **AS034.1 21d** | 18,59 ± 0,04 | 21,62 ± 0,31 | 19,08 ± 0,30 | - | 16,47 ± 0,23 |
| **SA121 4d** | 21,59 ± 0,18 | 22,39 ± 0,15 | 16,54 ± 0,26 | 23,42 ± 0,17 | 29,54 ± 0,37 |
| **SA121 14d** | 23,98 ± 0,28 | 23,91 ± 0,01 | 17,52 ± 0,27 | 26,64 ± 0,34 | 22,60 ± 0,29 |
| **SA121 21d** | 24,08 ± 0,46 | 23,88 ± 0,65 | 17,86 ± 0,43 | 26,63 ± 0,30 | 20,73 ± 0,60 |

| hBS cells | Nestin | Desmin | OC90 | Lin28 | Sox-2 | Gdf-3 | β-III-tubulin |
|---|---|---|---|---|---|---|---|
| **SA001 5d** | - | - | - | - | 19,36 ± 0,09 | 22,62 0,10 | 20,42 ± 0,52 |
| **SA001 14d** | **-** | - | - | - | 18,85 ± 0,14 | 23,66 ± 0,06 | 21,8 ± 0,67 |
| SA00121d | - | - | - | - | 19,81 ± 0,37 | 23,83 ± 0,66 | 21,96 ± 0,10 |
| **SA002 5d** | - | - | - | - | 19,02 ± 0,74 | 23,1 ± 0,94 | 21,07 ± 0,97 |
| **SA002 14d** | - | - | - | - | 19.16 ⊥ 0,47 | 23,87 ± 0,14 | 20,36 ± 0,45 |
| **SA002 21d** | - | - | - | - | 19,17 ± 0,67 | | 20,98 ± 0,39 |
| AS034 5d | - | - | - | - | 18,32 ⊥ 0,27 | 21,73 ± 0,09 | 20,01 ± 0,37 |
| AS03414d | - | - | - | - | 19,91 ± 0,10 | 23,74 ± 0,08 | 22,73 + 0,11 |
| **AS034 21d** | - | - | - | . | 20,37 ± 0,38 | 23,86 ± 0,14 | 22,46 ± 0,38 |
| AS034.1 4d | **-** | - | - | - | 20,83 ± 0,40 | 25,1 ± 0,41 | 22,76 ± 0,72 |
| **AS034.1 14d** | - | - | - | - | 19.54 ± 0,15 | 22,75 ± 0,13 | 22, 08 ± 0,41 |
| **AS034.1 21d** | - | - | - | - | 20,35 + 0,35 | 24,25 ± 0,43 | 22,85 ± 0,18 |
| **SA121 4d** | 24,81 ± 0,33 | 29,04 ⊥ 0,54 | 27,79 ± 0,55 | 16,9 ± 0,49 | 18,73 ± 0,38 | 22,53 ± 0,04 | 19,48 ± 0,36 |
| **SA121 14d** | 25,35 ± 0,16 | 28,46 ± 0,04 | 24,41 ± 0,21 | 17,33 ± 0,08 | 18,41 ± 0,33 | 23,75 ± 0,09 | 21,85 ± 0,05 |
| SA121 21d | 25,22 ± 0,13 | 28,63 ± 0,29 | 28,58 ± 1,08 | 17,40 ± 0,20 | 18,62 ± 0,17 | 23,43 ± 0,55 | 22,13 ± 0,29 |

The Ct value of Oct-4, Nanog, Cripto, DNMT3B, AFP, Nestin, Desmin OC90 and Un28 for SA121 is the mean value (±S.D) of one PCR reaction from 2 separate RT reactions.

For all other samples it is the mean value (±S.D) of one PCR reaction in duplicate from 2 separate RT reactions.

Plotting Ct (figure 5) shows that Oct-4, Nanog, and Cripto are genes that are expressed in undifferentiated hBS cells (low Ct), whereas they are down regulated upon differentiation (high Ct). It also shows that AFP is significantly up regulated in differentiated hBS cells compared to undifferentiated hBS cells. DNMNT3 is also expressed by undifferentiated hBS cells and is down regulated in differentiating hBS cells (see hBS cell line SA121).

The genes indicated above are all suggested to be markers for undifferentiated hBS cells or their differentiated progenies. Based on the results from the PCR analysis (see Ct-values) it is obvious that some genes are better suited as reporter genes than others. A reporter gene should have the following basic criteria: a) It should be either up or down regulated upon differentiation of the hBS cells and b) it should display a similar expression profile in several (e.g. more than 2) different hBS cell lines. Furthermore, when selecting a panel of reporter genes it is of importance that the combination of genes is chosen appropriately in order to maximize the sensitivity of the assay.

**Reference example 10**

**Quantifying the differentiation state of hBS cells**

In order to obtain a quantitative measure of the relative level of differentiation of the hBS cells, the mathematical model (Eq. 1) was applied. The input in the equation is the Ct-values for each individual reporter gene (Oct-4, Nanog, Cripto, and AFP) and the corresponding PCR-efficiency, and the output is an index based on the geometric averages between the RNA levels of down- and up regulated genes. By including several genes, the assay becomes very robust, while maintaining a high precision, and less sensitive to minor fluctuations in the expression levels of the individual genes. Figure 6 shows the calculated expression indexes from the QPCR analyses of hBS cells maintained on EF cells. For the five cell lines tested, the expression indexes ranged between 150 and 350 for undifferentiated hBS cells (day 4-5) and they were between 0.6 and 10 for differentiating hBS cells at day 14. In differentiated progenies from hBS cells at day 21, the expression indexes were further decreased and ranged between 0.05 and 2. The quantitatively most striking changes in the expression indexes occurred during the first week of differentiation. The fold-changes observed in the expression indexes were between 26 and 415 when comparing cells at day 5 and 14, and the fold-changes ranged between 1.2 and 15 when comparing cells at day 14 and 21.

Here we show that the combination of Cripto and AFP is clearly sufficient to discriminate between undifferentiated hBS cells and early derivatives thereof. Other combinations of potential reporter genes do not provide the same sensitivity with highest ratio in the summary table below.

*Two-gene ratios compared at day 4-5 and day 14.*

The tables below show how many times higher the two-gene index is for undifferentiated material (4-5 days in culture) compared to after 14 days in culture. The last 5 genes (nestin, desmin, OC90, Lin 28, DNMT3B ) were only tested on one of the cell lines. Of the tested genes Beta-III-tubulin, nestin (both ectodermal) and desmin (mesodermal) are genes that genes that are being up-regulated according to the literature searches performed, whereas Gdf-3, OC90, Lin28 and DNM3TB are genes that are being down-regulated according to the literature searches (normally expressed in undifferentiated hESCs). Although, no combination is superior in all cell lines, AFP and Cripto is the combination generating the over-all highest index ratio. The diagonal values 1.0 are obtained by inserting e.g. AFP both in the numerator and the denominator, whereby the index both at day 5 and day 14 is 1.

| SA001 p17 | AFP | Cripto | Sox-2 | Oct-4 | Nanog | Beta-III-tubulin | Gdf-3 |
|---|---|---|---|---|---|---|---|
| AFP | 1,0 | | | | | | |
| Cripto | 77,0 | 1,0 | | | | | |
| Sox-2 | 94,1 | 0,5 | 1,0 | | | | |
| Oct-4 | 94,1 | 1,2 | 2,3 | 1,0 | | | |
| Nanog | 75,3 | 1,0 | 1,8 | 0,8 | 1,0 | | |
| Beta-III-tubulin | 139,9 | 1,8 | 3,4 | 1,5 | 1,9 | 1,0 | |
| Gdf-3 | 111,9 | 1,5 | 2,7 | 1,2 | 1,5 | 0,8 | 1,0 |

| SA002 p156 | AFP | Cripto | Sox-2 | Oct-4 | Nanog | Beta-III-tubulin | Gdf-3 |
|---|---|---|---|---|---|---|---|
| AFP | 1,0 | | | | | | |
| Cripto | 23,1 | 1,0 | | | | | |
| Sox-2 | 3,3 | 0,1 | 1,0 | | | | |
| Oct-4 | 19,0 | 0,8 | 5,7 | 1,0 | | | |
| Nanog | 23,2 | 1,0 | 6,9 | 1,2 | 1,0 | | |
| Beta-III-tubulin | 1,9 | 0,1 | 0,6 | 0,1 | 0,1 | 1,0 | |
| Gdf-3 | 5,0 | 0,2 | 1,5 | 0,3 | 0,2 | 2,6 | 1,0 |

| AS034 p129 | AFP | Cripto | Sox-2 | Oct-4 | Nanog | Beta-III-tubulin | Gdf-3 |
|---|---|---|---|---|---|---|---|
| AFP | | | | | | | |
| Cripto | 967,1 | | | | | | |
| Sox-2 | 90,7 | 0,1 | | | | | |
| Oct-4 | 339,1 | 0,4 | 3,7 | | | | |
| Nanog | 224,5 | 0,2 | 2,5 | 0,7 | | | |
| Beta-III-tubulin | 187,0 | 0,2 | 2,1 | 0,6 | 0,8 | | |
| Gdf-3 | 118,0 | 0,1 | 1,3 | 0,3 | 0,5 | 0,6 | |

| AS034.1 p53 | AFP | Cripto | Sox-2 | Oct-4 | Nanog | Beta-III-tubulin | Gdf-3 |
|---|---|---|---|---|---|---|---|
| AFP | | | | | | | |
| Cripto | 157,8 | | | | | | |
| Sox-2 | 74,1 | 0,5 | | | | | |
| Oct-4 | 112,4 | 0,7 | 1,5 | | | | |
| Nanog | 108,0 | 0,7 | 1,5 | 1,0 | | | |
| Beta-III-tubulin | 109,2 | 0,7 | 1,5 | 1,0 | 1,0 | | |
| Gdf-3 | 37,4 | 0,2 | 0,5 | 0,3 | 0,3 | 0,3 | |

| SA121 p84 | AFP | Cripto | Sox | Oct | Nanog | Beta-III-tubulin |
|---|---|---|---|---|---|---|
| AFP | 1,0 | | | | | |
| Cripto | 175,9 | 1,0 | | | | |
| Sox-2 | 76,6 | 0,4 | 1,0 | | | |
| Oct-4 | 269.2 | 1,5 | 3,5 | 1,0 | | |
| Nanog | 214,3 | 1,2 | 2,8 | 0,8 | 1,0 | |
| Beta-III-tubulin | 431,4 | 2,5 | 5,6 | 1,6 | 2,0 | 1,0 |
| Gdf-3 | 205,9 | 1,2 | 2,7 | 0,8 | 1,0 0,5 | 0,5 N/A |
| NESTIN | 122,0 | 0,8 | N/A | 0,3 | | |
| DESMIN | 59,5 | 0,4 | N/A | 0,1 | 0,3 | N/A |
| OC90 | 9,8 | 0,1 | N/A | 0,0 | 0,0 | N/A |
| Lin28 | 112,3 | 0,7 | N/A | 0,3 | 0,5 | N/A |
| DNMT3B | 681,6 | 4,2 | N/A | 1,7 | 3,0 | N/A |

| SA121 p84 | Gdf | NESTIN | DESMIN | OC90 | Lin28 | DNMT3B |
|---|---|---|---|---|---|---|
| Gdf-3 | 1,0 | | | | | |

(continued)

| SA121 p84 | Gdf | NESTIN | DESMIN | OC90 | Lin28 | DNMT3B |
|---|---|---|---|---|---|---|
| **NESTIN** | N/A | 1,0 | | | | |
| **DESMIN** | N/A | 0.5 | 1,0 | | | |
| **OC90** | N/A | 0,1 | 0,2 | 1,0 | | |
| **Lin28** | N/A | 0,9 | 1,9 | 11,4 | 1,0 | |
| **DNMT3B** | N/A | 5,6 | 11,5 | 69,4 | 6,1 | 1,0 |

| Average | AFP | Cripto | Sox | Oct | Nanog | Beta-III-tubulin |
|---|---|---|---|---|---|---|
| **AFP** | 1,0 | | | | | |
| **Cripto** | 280,2 | 1,0 | | | | |
| **Sox-2** | 67,8 | 0,3 | 1,0 | | | |
| **Oct-4** | 166,8 | 0,9 | 3,4 | 1,0 | | |
| **Nanog** | 129,1 | 0,8 | 3,1 | 0,9 | 1,0 | |
| **Beta-III-tubulin** | 173,9 | 1,0 | 2,6 | 0,9 | 1,2 | 1,0 |
| **Gdf-3** | 95,6 | 0,6 | 1,7 | 0,6 | 0,7 | 1,0 |
| **NESTIN** | 122,0 | 0,8 | N/A | 0,3 | 0,5 | N/A |
| **DESMIN** | 59,5 | 0,4 | N/A | 0,1 | 0,3 | N/A |
| **OC90** | 9,8 | 0,1 | N/A | 0,0 | 0,0 | N/A |
| **Lin28** | 112,3 | 0,7 | N/A | 0,3 | 0,5 | N/A |
| **DNMT3B** | 681,6 | 4,2 | N/A | 1,7 | 3,0 | N/A |

| Average | Gdf | NESTIN | DESMIN | OC90 | Lin28 | DNMT3B |
|---|---|---|---|---|---|---|
| **Gdf-3** | 1,0 | | | | | |
| **NESTIN** | N/A | 1,0 | | | | |
| **DESMIN** | N/A | 0,5 | 1,0 | | | |
| **OC90** | N/A | 0,1 | 0,2 | 1,0 | | |
| **Lin28** | N/A | 0,9 | 1,9 | 11,4 | 1,0 | |
| **DNMT3B** | N/A | 5,6 | 11,5 | 69,4 | 6,1 | 1,0 |

*Three-gene ratios compared at day 4-5 and day 14 :*

The tables below show how many times higher the three-gene ratios are at day 4-5 compared to day 14 for the five different cell lines by extending the two gene ratios generated as above for the combination AFP and Cripto (Shown in the last column of each table). Apart from DNMT3B which was measured only in one cell line it is shown that Oct-4 seems to give the best result compared to the other genes presented.

| SA001 p17 | AFP | Cripto | Sox-2 | Oct-4 | Nanog | Beta-III-tubulin | Gdf-3 |
|---|---|---|---|---|---|---|---|
| AFP | | | | | | | |
| Cripto | 77,0 | | | | | | |
| Sox-2 | 94,1 | 0,5 | | | | | |
| Oct-4 | 94,1 | 1,2 | 2,3 | | | | |
| Nanog | 75,3 | 1,0 | 1,8 | 0,8 | | | |
| Beta-III-tubulin | 139,9 | 1,8 | 3,4 | 1,5 | 1,9 | | |
| Gdf-3 | 111,9 | 1,5 | 2,7 | 1,2 | 1,5 | 0,8 | |

| AFP/Cripto | | | 56,4 | 85,1 | 76,1 | 103,8 | 92,8 |
|---|---|---|---|---|---|---|---|

| SA002 p156 | AFP | Cripto | Sox-2 | Oct-4 | Nanog | Beta-III-tubulin | Gdf-3 |
|---|---|---|---|---|---|---|---|
| AFP | | | | | | | |
| Cripto | 23,1 | | | | | | |
| Sox-2 | 3,3 | 0,1 | | | | | |
| Oct-4 | 19,0 | 0,8 | 5,7 | | | | |
| Nanog | 23,2 | 1,0 | 6,9 | 1,2 | | | |

| Beta-III-tubulin | 1,9 | 0,1 | 0,6 | 0,1 | 0,1 | | |
|---|---|---|---|---|---|---|---|
| Gdf-3 | 5,0 | 0,2 | 1,5 | 0,3 | 0,2 | 2,6 | |

| AFP/Cripto | | | 8,8 | 21,0 | 23,1 | 6,7 | 10,8 |
|---|---|---|---|---|---|---|---|

| AS034 p129 | AFP | Cripto | Sox-2 | Oct-4 | Nanog | Beta-III-tubulin | Gdf-3 |
|---|---|---|---|---|---|---|---|
| AFP | | | | | | | |
| Cripto | 967,1 | | | | | | |
| Sox-2 | 90,7 | 0,1 | | | | | |
| Oct-4 | 339,1 | 0,4 | 3,7 | | | | |
| Nanog | 224,5 | 0,2 | 2,5 | 0,7 | | | |
| Beta-III-tubulin | 187,0 | 0,2 | 2,1 | 0,6 | 0,8 | | |
| Gdf-3 | 118,0 | 0,1 | 1,3 | 0,3 | 0,5 | 0,6 | |

| AFP/Cripto | | | 296,1 | 572,7 | 466,0 | 425,3 | 337,8 |
|---|---|---|---|---|---|---|---|

| AS034.1 p153 | AFP | Cripto | Sox-2 | Oct-4 | Nanog | Beta-III-tubulin | Gdf-3 |
|---|---|---|---|---|---|---|---|
| AFP | 1,0 | | | | | | |
| Cripto | 157,8 | 1.0 | | | | | |
| Sox-2 | 74,1 | 0,5 | | | | | |
| Oct-4 | 112,4 | 0,7 | 1,5 | 1,0 | | | |
| Nanog | 108,0 | 0,7 | 1,5 | 1,0 | 1,0 | | |
| Beta-III-tubulin | 109,2 | 0,7 | 1,5 | 1,0 | 1,0 | 1,0 | |
| Gdf-3 | 37,4 | 0,2 | 0,5 | 0,3 | 0,3 | 0,3 | 1,0 |

| AFP/Cripto | | | 108,1 | 133,2 | 130,5 | 131,3 | 76,8 |
|---|---|---|---|---|---|---|---|

| SA121 p84 | AFP | Cripto | Sox-2 | Oct-4 | Nanog | Beta-III-tubulin |
|---|---|---|---|---|---|---|
| AFP | 1,0 | | | | | |
| Cripto | 175,9 | 1.0 | | | | |
| Sox-2 | 76,6 | 0,4 | 1,0 | | | |
| Oct-4 | 269,2 | 1,5 | 3,5 | 1,0 | | |
| Nanog | 214,3 | 1,2 | 2,8 | 0,8 | 1,0 | |
| Beta-III-tubulin | 431,4 | 2,5 | 5,6 | 1,6 | 2,0 | 1,0 |
| Gdf-3 | 205,9 | 1,2 | 2,7 | 0,8 | 1,0 | 0.5 |
| NESTIN | 122,0 | 0,8 | N/A | 0,3 | 0,5 | N/A |
| DESMIN | 59,5 | 0,4 | N/A | 0,1 | 0,3 | N/A |
| OC90 | 9,8 | 0,1 | N/A | 0,0 | 0,0 | N/A |
| Lin28 | 112,3 | 0,7 | N/A | 0,3 | 0,5 | N/A |
| DNMT3B | 681,6 | 4,2 | N/A | 1,7 | 3,0 | N/A |
| | | | | | | |
| AFP/ Cripto | | | 116,1 | 217,6 | 194,2 | 275,5 |

| SA121 p84 | Gdf-3 | NESTIN | DESMIN | OC90 | Lin28 | DNMT3B |
|---|---|---|---|---|---|---|
| Gdf-3 | 1,0 | | | | | |
| NESTIN | N/A | 1,0 | | | | |
| DESMIN | N/A | 0,5 | 1,0 | | | |
| OC90 | N/A | 0,1 | 0,2 | 1,0 | | |
| Lin28 | N/A | 0,9 | 1,9 | 11,4 | 1,0 | |

(continued)

| SA121 p84 | Gdf-3 | NESTIN | DESMIN | OC90 | Lin28 | DNMT3B |
|---|---|---|---|---|---|---|
| DNMT3B | N/A | 5,6 | 11,5 | 69,4 | 6,1 | 1,0 |
| | | | | | | |
| AFP/ Cripto | 190,3 | 140,3 | 97,9 | 39,8 | 134,6 | 331,6 |

| Average | AFP | Cripto | Sox-2 | Oct-4 | Nanog | Beta-III-tubulin |
|---|---|---|---|---|---|---|
| AFP | 1,0 | | | | | |
| Cripto | 280,2 | 1,0 | | | | |
| Sox-2 | 67,8 | 0,3 | 1,0 | | | |
| Oct-4 | 166,8 | 0.9 | 3,4 | 1,0 | | |
| Nanog | 129,1 | 0,8 | 3,1 | 0,9 | 1,0 | |
| Beta-III-tubulin | 173,9 | 1,0 | 2,6 | 0,9 | 1,2 | 1,0 |
| Gdf-3 | 95,6 | 0,6 | 1,7 | 0,6 | 0,7 | 1,0 |
| NESTIN | 122,0 | 0,8 | N/A | 0,3 | 0,5 | N/A |
| DESMIN | 59,5 | 0,4 | N/A | 0,1 | 0,3 | N/A |
| OC90 | 9,8 | 0,1 | N/A | 0,0 | 0,0 | N/A |
| Lin28 | 112,3 | 0,7 | N/A | 0,3 | 0,5 | N/A |
| DNMT3B | 681,6 | 4,2 | N/A | 1,7 | 3,0 | N/A |
| | | | | | | |
| AFP/ Cripto | | | 117,1 | 205,9 | 178,0 | 188,5 |

| Average | Gdf-3 | NESTIN | DESMIN | OC90 | Lin28 | DNMT3B |
|---|---|---|---|---|---|---|
| Gdf-3 | 1,0 | | | | | |
| NESTIN | N/A | 1,0 | | | | |
| DESMIN | N/A | 0,5 | 1,0 | | | |
| OC90 | N/A | 0,1 | 0,2 | 1,0 | | |
| Lin28 | N/A | 0,9 | 1,9 | 11,4 | 1,0 | |
| DNMT3B | N/A | 5,6 | 11,5 | 69,4 | 6,1 | 1,0 |
| | | | | | | |
| AFP/ Cripto | 141,7 | 140,3 | 97,9 | 49,6 | 67,5 | 331,6 |

The calculations as presented in this example can of course be further extended to include even more reporter genes. If such reporter genes are selected arbitrarily without prior testing there is a significant risk that the sensitivity and robustness of the assay is compromised.

**Reference example 11**

**Quantifying the differentiation state of hBS cells separated into SSEA-4 positive and negative fractions**

hBS cells were cultured as in example 1 for 7 days without passaging. Almost 300 colonies that by visual inspection appeared both morphologically undifferentiated and differentiated were cut out mechanically, using a stem cell cutting tool (Swemed Labs International, Billdal, Sweden), as whole colonies. The colonies were dissociated in 0.5 mM EDTA for 30 minutes into a single cell suspension and after centrifugation resuspended in PBS, (containing 0.1 % BSA), to a final concentration of 100 000 cells/ml. The cell suspension were mixed with SSEA-4 coated magnetic Dynabeads (CELLection Pan Mouse IgG, Dynal, Norway) in a cell to bead ratio of 1:25 and incubated for 1h at +4°C. The SSEA-4 positive cells bound to the beads were separated from the negative cells in a magnetic stand, and washed in PBS (containing 0, 1 % BSA). Positive and negative cell fractions were stored in -80°C. RNA extraction, reverse transcription, QPCR, and quantifications were performed as described in the examples above.

Approximately 60% of the cells were captured by the SSEA-4-antibody coated beads. The results from the QPCR analysis are shown in Figure 7 and demonstrate that the expression index of SSEA-4+ cells is about 15-folds higher than the expression index of SSEA-4- cells.

## Claims

1. A kit for use in real-time PCR to assess the state of hBS cell differentiation comprising a panel comprising four pairs of primers, which are complementary to the reporter genes AFP, Cripto, Oct-4 and Nanog.

2. A kit according to claim 1 comprising:

    i) a panel of primers as defined in claim 1,
    ii) optionally a suitable PCR mix,
    iii) optionally one or more components for reverse transcription,
    iv) optionally, instructions for use thereof.

## Patentansprüche

1. Kit zur Verwendung in Real-Time PCR zur Bewertung des Zustands einer hBS-Zelldifferenzierung, umfassend ein Panel umfassend vier Paare von Primern, welche zu den Reportergenen AFP, Cripto, Oct-4 und Nanog komplementär sind.

2. Kit nach Anspruch 1, umfassend:

    i) ein Panel von Primern wie in Anspruch 1 definiert,
    ii) wahlweise ein geeignetes PCR-Gemisch,
    iii) wahlweise eine oder mehrere Komponenten für die reverse Transkription,
    iv) wahlweise Gebrauchsanweisungen dafür.

## Revendications

1. Kit pour l'utilisation dans la PCR en temps réel pour l'évaluation de l'état de la différenciation de cellules hBS comprenant un panel comprenant quatre paires d'amorces qui sont complémentaires aux gènes rapporteurs AFP, Cripto, Oct-4 et Nanog.

2. Kit selon la revendication 1 comprenant:

    i) un panel d'amorces tel que défini dans la revendication 1,
    ii) éventuellement un mélange de PCR approprié,
    iii) éventuellement un ou plusieurs composants pour la transcription inverse,
    iv) le cas échéant, des instructions pour l'utilisation de ceux-ci.

**Fig. 1**

**Fig. 2**

Fig. 3

**Fig. 4**

**Fig. 5**

Fig. 6

**Fig. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030224411 A
- WO 03055992 A